(19) Europäisches Patentamt
European Patent Office
Office européen des brevets

(11) **EP 2 662 020 A1**

(12) **EUROPEAN PATENT APPLICATION**

(43) Date of publication:
**13.11.2013 Bulletin 2013/46**

(51) Int Cl.:
**A61B 5/053** (2006.01)   **A61B 5/00** (2006.01)

(21) Application number: **13001338.6**

(22) Date of filing: **15.03.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.05.2012 JP 2012108907**

(71) Applicant: **Tanita Corporation
Tokyo 174-8630 (JP)**

(72) Inventors:
• **Uchiyama, Tomoka
  Tokyo, 174-8630 (JP)**

• **Fukuda, Risa
  Kyoto-shi, Kyoto, 606-8507 (JP)**
• **Arakawa, Chitoso
  Hikone-city, Shiga, 522-8533 (JP)**
• **Akazawa, Chiharu
  Kyoto-shi, Kyoto, 606-8507 (JP)**

(74) Representative: **Müller-Boré & Partner
Patentanwälte
Grafinger Straße 2
81671 München (DE)**

(54) **Edema evaluation apparatus**

(57)     An index calculating section calculates an edema index value indicating the level of edema at a target portion of the body of a human subject, according to a bioelectric impedance of the target portion. An edema evaluation section identifies the edema stage corresponding to the edema index value calculated by the index calculating section from among a plurality of edema stages indicating the degrees of progress of edema symptoms. A result reporting section reports the edema stage identified by the edema evaluation section to the user.

FIG. 1

## Description

### Technical Field

The present invention relates to technologies for evaluating the level of edema produced in the body of a human subject.

### Background Art

To manage edema such as lymphatic edema (in medical management or in self-care), it is necessary to quantitatively recognize the level of edema. It is possible to estimate the level of edema by measuring the circumferences of the four limbs of a human subject, but accurate and objective estimation is not easy. Taking these situations into account, technologies have been proposed in which it is determined whether edema occurs at a specific portion of the body by using the measured value of bioelectric impedance of the body of a human subject. For example, Patent Document 1 discloses a technology in which the ratio of the bioelectric impedance of an affected limb of a human subject to the bioelectric impedance of a healthy limb is used as an edema index, and information about the existence of edema (whether the edema index is within a normal range) is provided to a user.

Citation List

### Patent Document

Patent Document 1: Japan Patent Application Publication JP-2010-526604-A

### Summary of Invention

### Technical Problem

In the technology of Patent Document 1, however, since only the existence of edema is reported, it is not easy to recognize how edema produced in the body of a human subject has progressed. Taking the above-described situations into consideration, an object of the present invention is to allow a user to easily recognize the degree of progress of edema produced in the body of a human subject.

### Solution to Problem

An edema evaluation apparatus according to the present invention includes index calculator for calculating an edema index value indicating the level of edema at a target portion of the body of a human subject, according to a bioelectric impedance of the target portion, edema evaluator for identifying an edema stage corresponding to the edema index value calculated by the index calculator from among a plurality of edema stages indicating the degrees of progress of edema symptoms, and result reporter for reporting the edema stage identified by the edema evaluator. In the above configuration, the edema stage corresponding to the edema index value in the plurality of edema stages, which indicate the degrees of progress of the edema, is reported. Therefore, the user can easily recognize the degree of progress of the edema of the human subject.

It is preferable that the result reporter further report the edema index value calculated by the index calculator. Since the edema index value is reported together with the edema stage, the user can recognize the level of edema within one edema stage.

It is preferable that the index calculator calculate the edema index value according to a discrepancy between a body composition index value corresponding to a bioelectric impedance of a right limb and a body composition index value corresponding to a bioelectric impedance of a left limb of the human subject. Since the edema index value is calculated according to the discrepancy between the body composition index value of the right limb and the body composition index value of the left limb, the user can easily recognize the difference in the degree of progress of the edema between the right and left limbs. In addition, in a configuration in which the index calculator calculates the edema index value by dividing the difference between the body composition index value of the right limb and the body composition index value of the left limb of the human subject by a body frame index value of the human subject, differences in body frame (weight) among human subjects are corrected for and the level of edema can be accurately evaluated.

It is preferable that the index calculator can execute a first process in which an edema index value corresponding to a bioelectric impedance of a right limb and an edema index value corresponding to a bioelectric impedance of a left limb of the human subject are individually calculated, and a second process in which an edema index value is calculated according to a discrepancy between a body composition index value corresponding to a bioelectric impedance of a right limb and a body composition index value corresponding to a bioelectric impedance of a left limb of the human subject; and, when edema occurs in both the right limb and the left limb of the human subject, the edema evaluator individually identify, for each of the right and left limbs, the edema stage corresponding to the edema index value calculated in the first process, and when edema occurs in either the right limb or the left limb, the edema evaluator identify the edema stage corresponding to the edema index value calculated in the second process. When edema occurs in both a right limb and a left limb of the human subject, the edema stage corresponding to the edema index value is identified individually for each of the right and left limbs, and, when edema occurs in either a right limb or a left limb of the human subject, the edema stage is identified from the edema index value based on the discrepancy in body composition index value between the right and left limbs. Therefore, an advantage is provided in that the edema of the human subject can be appropriately evaluated for either a case in which edema occurs in both a right limb and a left limb or a case in which edema occurs in either a right limb or a left limb. It is preferable that the index calculator determine whether edema occurs in both the right limb and the left limb of the human subject, according to the edema index values calculated in the first process for the right and left limbs, and, when edema occurs in either the right limb or the left limb, the index calculator execute the second process.

An edema evaluation apparatus according to the present invention is implemented with the cooperation of a general-purpose computational processor, such as a central processing unit (CPU), and a program. A program according to the present invention causes a computer to execute an index calculating process of calculating an edema index value indicating the level of edema at a target portion of the body of a human subject, according to a bioelectric impedance of the target portion, an edema evaluation process of identifying an edema stage corresponding to the edema index value calculated in the index calculating process from among a plurality of edema stages indicating the degrees of progress of edema symptoms, and a result reporting process of reporting the edema stage identified in the edema evaluation process. The program of the present invention is stored in a computer-readable recording medium, is provided in that form, and is installed in a computer. Alternatively, the program of the present invention is distributed through a communication network and is installed in a computer.

**Brief Description of Drawings**

FIG. 1 is a block diagram of an edema evaluation apparatus according to a first embodiment of the present invention.
FIG. 2 is a block diagram of an index calculating section.
FIG. 3 is a graph showing the relationship between an edema stage and an edema index value.
FIG. 4 is a table explaining the relationship between the edema stage and  the edema index value.
FIG. 5 is a typical view of an evaluation-result image.
FIG. 6 is a graph showing the relationship between an edema stage and an edema index value in a second embodiment.
FIG. 7 is a flowchart of edema evaluation processing in a third embodiment.
FIG. 8 is a typical view of an evaluation-result image in a modification.

**Description of Embodiments**

**First embodiment**

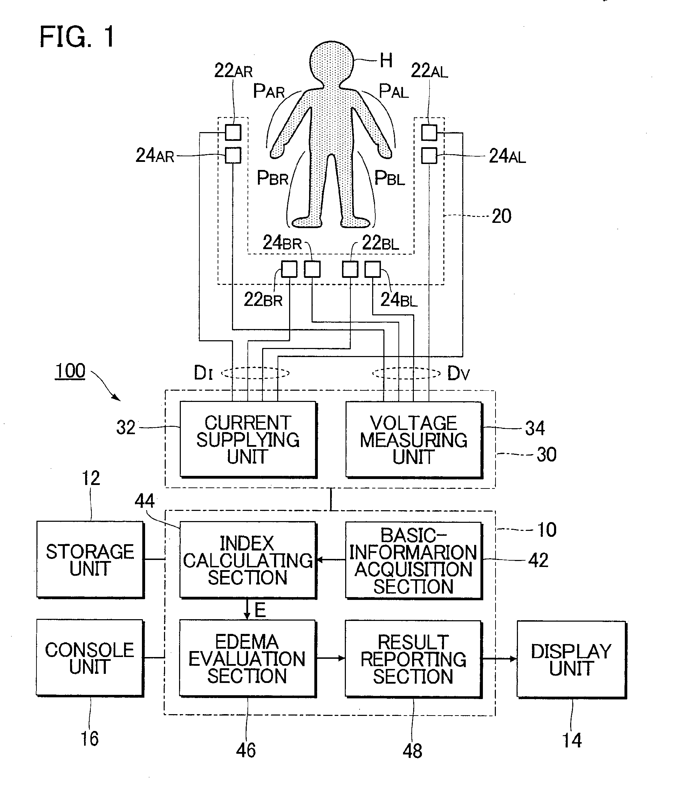

FIG. 1 is a block diagram of an edema evaluation apparatus 100 according to a first embodiment of the present invention. The edema evaluation apparatus 100 shown in FIG. 1 is a biomedical measurement apparatus for evaluating the level of edema produced in the body of a human subject H. It is assumed in the first embodiment that lymphatic edema in the human subject H is evaluated. Lymphatic edema is produced when the flow of lymph is blocked or reduced due to stenosis or obstruction in a lymphatic vessel or a lymph node. As shown in FIG. 1, the edema evaluation apparatus 100 according to the first embodiment includes a controller 10, a storage unit 12, a display unit 14, a console unit 16, a group of electrodes 20, and an electrode controller 30.

The controller 10 (CPU) executes a program stored in the storage unit 12 to control each part of the edema evaluation apparatus 100. The storage unit  12 is a storage circuit (such as a ROM or a RAM) for storing the program executed by the controller 10 and various types of data used by the controller 10. The display unit 14 (for example, a liquid crystal display panel) displays various types of images under the control of the controller 10. For example, the display unit 14 displays an evaluation result (level of edema) determined by the edema evaluation apparatus 100.

The console unit 16 is an input unit for receiving an instruction from a user (the human subject H or an operator), and includes, for example, a plurality of manipulanda operable by the user. The user can appropriately operate the console unit 16 to specify a specific portion (hereafter called a target portion) in which edema is to be evaluated, in the body of the human subject H. In the first embodiment, in the body of the human subject H, one of the upper right limb (right arm) PAR, the upper left limb (left arm) PAL, the lower right limb (right leg) PBR, and the lower left limb (left leg) is specified as a target portion.

The group of electrodes 20 includes a plurality of current supplying electrodes 22 (22AR, 22AL, 22BR, and 22BL) and a plurality of voltage measuring electrodes 24 (24AR, 24AL, 24BR, and 24BL), and is used to measure the bioelectric impedance of the body of the human subject H. The current supplying electrode 22AR and the voltage measuring electrode 24AR are placed in contact with the right hand (palm), and the current supplying electrode 22AL and the voltage measuring electrode 24AL are placed in contact with the left hand (palm). The current supplying electrode 22BR and the voltage measuring electrode 24BR are placed in contact with the right foot (sole), and the current supplying electrode 22BL and the voltage measuring electrode 24BL are placed in contact with the left foot (sole).

The positions where the group of electrodes 20 are installed and the number of electrodes can be changed appropriately. When the edema evaluation apparatus 100 is mounted to a body composition metering apparatus provided with a base on which the human subject H stands and a handle gripped by the human subject H, the electrodes (22BR, 22BL, 24BR, and 24BL) for the lower limbs are placed on the base and the electrodes (22AR, 22AL, 24AR, and 24AL) for the upper limbs are installed on the handle. It is also possible that the group of electrodes 20 are attached to the surface of the body of the human subject H independently from each other. A grippable measuring apparatus (such as a bioelectric impedance measuring apparatus) in which a plurality of electrodes are arranged on a surface that contacts the body of the human subject H can measure the bioelectric impedance of a portion (target portion) in the body of the human subject H contacting the measuring apparatus.

The electrode controller 30 includes a current supplying unit 32 and a voltage measuring unit 34. The current supplying unit 32 supplies measuring current DI between a pair of current supplying electrodes 22 selected from the plurality of current supplying electrodes 22 of the group of electrodes 20. The measuring current DI is an alternating current having a predetermined frequency (for example, 5 kHz to 250 kHz), flowing through the body of the human subject H between the pair of current supplying electrodes 22. The voltage measuring unit 34 measures the voltage (hereafter called a measurement voltage) DV between a pair of voltage measuring electrodes 24 selected from the plurality of voltage measuring electrodes 24 of the group of electrodes 20. The measurement voltage DV is converted to a digital signal by an A/D converter (not shown) and is sent to the controller 10.

As shown in FIG. 1, the controller 10 executes the program stored in the storage unit 12 to function as a plurality of elements (a basic-information acquisition section 42, an index calculating section 44, an edema evaluation section 46, and a result reporting section 48) to evaluate edema in the body of the human subject H. It is also possible to distribute the functions of the controller 10 among a plurality of apparatuses. The basic-information acquisition section 42 acquires basic information related to the body of the human subject H. The basic-information acquisition section 42 acquires, for example, the height L of the human subject H, specified by the user with an operation on the console unit 16. It is also preferable that the basic-information acquisition section 42 acquire the height L of the human subject H measured by a measuring unit (not shown) or acquire the height L of the human subject H stored in the storage unit 12.

The index calculating section 44 calculates an edema index value E indicating the level of edema in the target portion of the body of the human subject H according to the bioelectric impedance of the target portion. For example, the index calculating section 44 calculates an edema index value E every time the user gives an instruction to start measurement on the console unit 16.

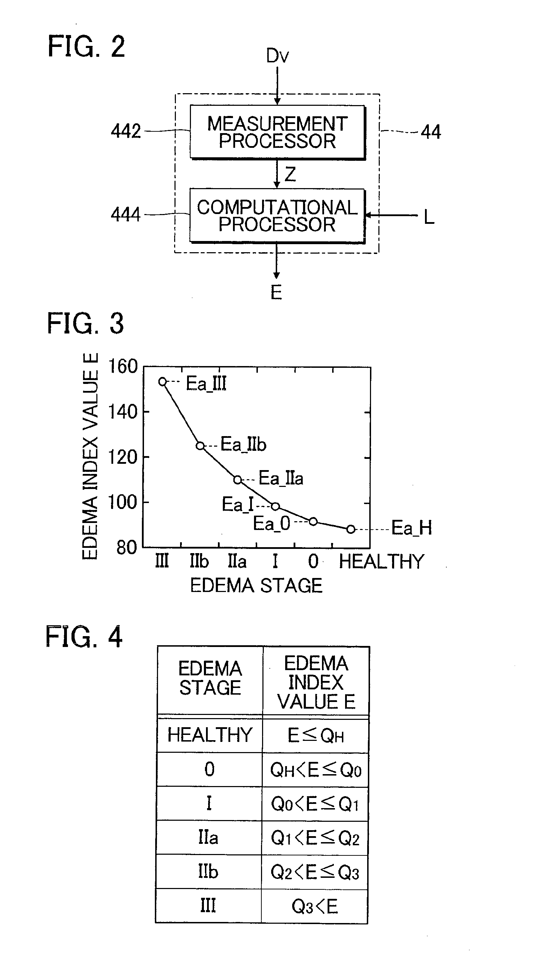

FIG. 2 is a block diagram of the index calculating section 44. As shown in FIG. 2, the index calculating section 44 includes a measurement processor 442 and an computational processor 444. The measurement processor 442 measures the bioelectric impedance Z (ZAR, ZAL, ZBR, or ZBL) of the target portion. Specifically, the measurement processor 442 measures the bioelectric impedance Z of the target portion from the relationship between the measurement current DI supplied from the current supplying unit 32 and the measurement voltage DV detected by the voltage measuring unit 34.

When the upper right limb PAR is specified as the target portion, for example, the measurement processor 442 controls the electrode controller 30 such that the measurement current DI is supplied between the current supplying electrode 22AR and the current supplying electrode 22BR (to a path that passes through the upper right limb PAR and the lower right limb PBR of the human subject H), and the measurement voltage DV between the voltage measuring electrode 24AR and the voltage measuring electrode 24AL (in a path that passes through the upper right limb PAR and the upper left limb PAL of the human subject H) is detected. Then, the measurement processor 442 calculates the bioelectric impedance ZAR of the upper right limb PAR from the relationship between the measurement current DI and the measurement voltage DV. In the same manner, the measurement processor 442 can calculate the bioelectric impedance ZAL of the upper left limb PAL, the bioelectric impedance ZBR of the lower right limb PBR, and the bioelectric impedance ZBL of the lower left limb PBL by selecting, depending on the target portion, a pair of current supplying electrodes 22 that supply the measurement current DI and a pair of voltage measuring electrodes 24 that detect the measurement voltage DV.

The computational processor 444 shown in FIG. 2 calculates the edema index value E according to the bioelectric impedance Z (ZAR, ZAL, ZBR, or ZBL) of the target portion calculated by the measurement processor 442. The edema index value E is a scale used to evaluate the level of edema (the amount of water) at the target portion. The edema index value E calculated by the computational processor 444 is stored in the storage unit 12. In other words, the time sequence (temporal changes) of the edema index values of the human subject H are stored in the storage unit 12.

The computational processor 444 calculates the edema index value (impedance index) E by the following expression (1) to which the height L of the human subject H acquired by the basic-information acquisition section and the bioelectric impedance Z of the target portion calculated by the measurement processor 442 are applied:

$$E = L^2/Z \qquad (1)$$

As understood from Expression (1), as the amount of water increases in the target portion due to the progress of the edema (that is, as the bioelectric impedance Z decreases), the edema index value E increases.

The following expression (2A) indicates the bioelectric impedance Z for a case in which it is assumed that a specific portion of the body of the human subject H is a cylinder having a cross section "a" and a length "h":

$$Z = \rho \cdot h/a \qquad (2A)$$

where p indicates the resistivity.

Expression (2A) is changed to the following expression (2B) when the right side of Expression (2A) is multiplied by h/h and the volume V (V = a·h) of the cylinder is introduced:

$$V = \rho(h)^2/Z \qquad (2B)$$

The volume V in Expression (2B) corresponds to the volume of tissue (hereafter called path tissue) serving, depending on the frequency of the measurement current DI, as the path of the measurement current DI in the target portion. Taking into account the tendency for the length H of the target portion to be almost proportional to the height L of the human subject H, it can be said that the edema index value E is an index for the volume of the path tissue, as understood from a comparison between Expression (1) and Expression (2B). Because the volume (Volume V) of the path tissue in the target portion increases as the amount of water increases in the target portion due to the progress of the edema, the edema index value E in Expression (1) increases as the edema progresses.

The edema evaluation section 46 shown in FIG. 1 identifies the edema stage corresponding to the edema index value E calculated by the index calculating section 44. The edema stage is a classification of the degree of progress of the edema. The edema evaluation section 46 identifies the edema stage corresponding to the edema index value E among a plurality of edema stages (0, I, IIa, IIb, and III) stipulated (in 2003) by the International Lymph Society. As the edema stage changes from 0 to III, the level of seriousness of the lymphatic edema increases. In the first embodiment, the human subject H can be evaluated as being in a state in which no edema occurs (hereafter called a healthy state) in addition to being in a certain edema stage.

Edema stage 0 is an early stage in which lymph circulation is insufficient but no clinical symptoms are observed (latent lymphatic edema); edema stage I is a stage in which slight swelling is observed but is improved when the affected portion is raised (reversible lymphatic edema); edema stage II (IIa and IIb) is a stage in which swelling is not improved even when the affected portion is raised (irreversible lymphatic edema), which is divided into an early edema stage IIa and a late edema stage IIb; and edema stage III is a stage in which a fiber network is formed due to the degeneration of protein in a tissue gap, and fat is hardened (elephantiasis).

FIG. 3 is a graph showing the average Ea (Ea_0, Ea_I, Ea_IIa, Ea_IIb, and Ea_III) of the edema index values E calculated for a plurality of edema patients clinically classified into the edema stages. It is understood from FIG. 3 that there is tendency that the larger the edema index value E is, the higher the edema stage is (the more the edema has progressed). FIG. 3 also shows the average Ea_H of the edema index values E calculated for healthy human subjects.

FIG. 4 shows the relationship between the edema index value E calculated by the index calculating section 44 and the edema stage identified by the edema evaluation section 46. The edema evaluation section 46 evaluates edema (identifies the edema stage) according to a plurality of reference values Q (QH, Q0, Q1, Q2, and Q3, where QH < Q0 < Q1 < Q2 < Q3). Specifically, as shown in FIG. 4, the edema evaluation section 46 determines that, when the edema index value E is equal to or smaller than the reference value QH (E ≤ QH), the human subject H is in a healthy condition; and, when the edema index value E is larger than the reference value QH, the human subject H has edema. More specifically, the edema evaluation section 46 determines that, when the edema index value E is larger than the reference value QH and equal to or smaller than the reference value Q0 (QH < E ≤ Q0), the human subject H is in edema stage 0; when the edema index value E is larger than the reference value Q0 and equal to or smaller than the reference value Q1 (Q0 < E ≤ Q1), the human subject H is in edema stage I; when the edema index value E is larger than the reference value Q1 and equal to or smaller than the reference value Q2 (Q1 < E ≤ Q2), the human subject H is in edema stage IIa; when the edema index value E is larger than the reference value Q2 and equal to or smaller than the reference value Q3 (Q2 < E ≤ Q3), the human subject H is in edema stage IIb; and, when the edema index value E is larger than the reference value Q3 (E > Q3), the human subject H is in edema stage III. In other words, the reference values Q are the boundary values between the edema stages arranged one after another (as well as the boundary value between the healthy condition and the edema stage 0).

The reference values Q are specified in advance, taking the relationship shown in FIG. 3 into account. Specifically, the middle value between the average Ea_H (the average of the edema index values E of human subjects in healthy conditions) and the average Ea_0 (the average of the edema index values E of a plurality of edema patients clinically diagnosed as being in edema stage 0), shown in FIG. 3, is used as the reference value QH; the middle value between the average Ea_0 (the average of the edema index values E of the plurality of edema patients clinically diagnosed as being in edema stage 0)and the average Ea_I (the average of the edema index values E of a plurality of edema patients clinically diagnosed as being in edema stage I), shown in FIG. 3, is used as the reference value Q0; the middle value between the average Ea_I and the average Ea_IIa is used as the reference value Q1; the middle value between the average Ea_IIa and the average Ea_IIb is used as the reference value Q2; and, the middle value between the average Ea_IIb and the average Ea_III is used as the reference value Q3. The reference values Q specified as described above are stored in the storage unit 12 and are used to identify the edema stage in the edema evaluation section 46.

How the reference values Q are specified is not limited to that described above. For example, it is possible that the standard deviation of the edema index values E of a plurality of edema patients is calculated in each edema stage, and, when the ranges in each of which the edema index values E are distributed according to the standard deviation (for example, the ranges each of which is specified by the average plus or minus the standard deviation) overlap between the edema stages arranged side by side, the middle value in the overlapping zone is used as the reference value Q at the boundary of the stages.

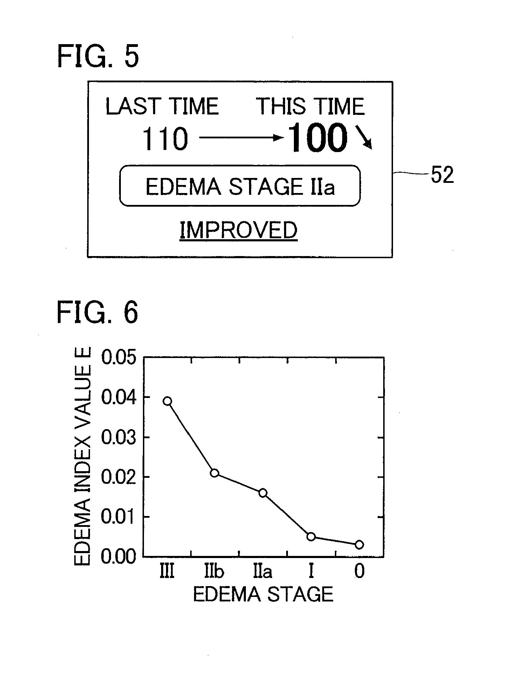

The result reporting section 48 shown in FIG. 1 reports the result of evaluation for the edema of the human subject H to the user. Specifically, the result reporting section 48 generates an evaluation result image 52 shown in FIG. 5 as an example image and displays it on the display unit 14. In the evaluation result image 52, the edema stage identified by the edema evaluation section 46 (in the example image in FIG. 5, edema stage IIa) is displayed, and the edema index value E calculated by the index calculating section 44 this time (in the example image in FIG. 5, 100) and the edema index value E calculated last time (in the example image in FIG. 5, 110) are also displayed in a manner allowing comparison. When the edema evaluation section 46 determines that the human subject H is in a healthy condition, a message indicating that fact is displayed on the display unit 14.

A message corresponding to the result of comparison between the edema index value E calculated this time and the edema index value E calculated last time is also indicated in the evaluation result image 52. Specifically, when the edema index value E is reduced from the evaluation last time, a message indicating improvement of the edema (in the example image in FIG. 5, "improved") is displayed; and, when the edema index value E is increased from the evaluation last time, a message indicating deterioration of the edema (for example, "deteriorated") is displayed. The distance from the edema index value E calculated this time and an edema stage adjacent to the current edema stage can be displayed. For example, when the human subject H is evaluated as being in edema stage IIa in the evaluation this time, the message "by δ to edema stage I" can be displayed. The value δ is set to the difference between the edema index value E calculated this time and the reference value Q between the current edema stage and an edema stage adjacent to the current edema stage (specifically, the edema stage closer to the edema index value E calculated this time).

As described above, in the first embodiment, the edema stage corresponding to the edema index value E, which indicates the degree of progress of the edema in the human subject H, in the plurality of edema stages is provided to the user. Therefore, the user can easily recognize the degree of progress of the edema that occurs in the target portion in the body of the human subject H.

The level of edema in the human subject H within one edema stage can vary. In the first embodiment, the edema stage identified by the edema evaluation section 46 and the edema index value E calculated by the index calculating section 44 are provided to the user. Therefore, the user can easily recognize the edema stage as described earlier, and, in addition, the user can recognize the level of edema in one edema stage (for example, whether the level of edema in edema stage IIa is closer to edema stage I or to edema stage IIb) by checking the edema index value E.

In the technology disclosed in Patent Document 1, the ratio of the bioelectric impedance between a right limb and a left limb of the human subject H is calculated as an index of edema. Therefore, if both the right limb and the left limb of the human subject H have edema (especially if the right limb and the left limb have edema at similar levels), the result would be the same as in a case in which both the right limb and the left limb have low levels of edema, and edema in the four limbs of the human subject H cannot be appropriately evaluated. In addition, the level of edema cannot be evaluated for a human subject H who lacks one of the right and left limbs among the four limbs. Conversely, in the first embodiment, since edema is evaluated for each of the right limb and the left limb of the human subject H, the level of edema can be appropriately recognized for a human subject H having edema in both the right and left limbs, or for a human subject H who lacks one of the four limbs.

Second embodiment

A second embodiment of the present invention will be described below. Note that, for units in the following example embodiments having the same structures or functions as in the first embodiment, the reference symbols used in the above description of the first embodiment will be used again, and detailed descriptions thereof will be omitted, if unnecessary.

The basic-information acquisition section 42 of the second embodiment acquires the weight W of the human subject H measured with scales (not shown). The basic-information acquisition section 42 may acquire the weight W input by the user through the console unit 16 or the weight W stored in the storage unit 12.

The index calculating section 44 (computational processor 444) calculates a body composition index value C (CAR, CAL, CBR, or CBL) according to the bioelectric impedance Z of each of the portions (four limbs) of the body of the human subject H. The body composition index value C is an index related to the body composition of each of the four limbs (arms and legs) of the human subject H (an index that changes according to the level of edema). In the second embodiment, the weight is used as the body composition index value C. Specifically, the body composition index value CAR calculated from the bioelectric impedance ZAR is the weight of the upper right limb PAR, the body composition index value CAL calculated from the bioelectric impedance ZAL is the weight of the upper left limb PAL, the body composition index value CBR calculated from the bioelectric impedance ZBR is the weight of the lower right limb PBR, and the body composition index value CBL calculated from the bioelectric impedance ZBL is the weight of the lower left limb PBL. Any known technique can be used to calculate the body composition index value C (estimate the weight) according to the bioelectric impedance.

The user can operate the console unit 16 appropriately to specify either the upper limbs (the upper right limb PAR and the upper left limb PAL) or the lower limbs (the lower right limb PBR and the lower left limb PBL) of the body of the human subject H as target portions. When the user specifies the upper limbs as target portions, the index calculating section 44 calculates the edema index value E by the following expression:

$$E = |CAR - CAL|/W \qquad (3A)$$

As understood from Expression (3A), the edema index value E is calculated according to the discrepancy between the body composition index value CAR of the upper right limb PAR and the body composition index value CAL of the upper left limb PAL (in Expression (3A), the absolute value of the difference |CAR - CAL|). Therefore, the larger the discrepancy between the body composition index value CAR and the body composition index value CAL, the larger the edema index value E. When edema is worse in one of the upper right limb PAR and the upper left limb PAL than in the other, the weight (body composition index value C) of the more affected portion increases as the amount of water therein increases. Therefore, the edema index value E in Expression (3A) increases as the edema progresses in one of the upper right limb PAR and the upper left limb PAL than in the other. The division by the weight W is to correct for differences in body frame among human subjects (to normalize the edema index value E).

In contrast, when the user specifies the lower limbs as target portions, the index calculating section 44 calculates the edema index value E by the following expression:

$$E = |CBR - CBL|/W \qquad (3B)$$

As understood from Expression (3B), the edema index value E is calculated according to the discrepancy between the body composition index value CBR of the lower right limb PBR and the body composition index value CBL of the lower left limb PBL (in Expression (3B), the absolute value of the difference |CBR - CBL|). Therefore, the larger the discrepancy between the body composition index value CBR and the body composition index value CBL, the larger the edema index value E. When edema is worse in one of the lower right limb PBR and the lower left limb PBL than in the other, the weight (body composition index value C) of the more affected portion increases as the amount of water therein increases.

Therefore, the edema index value E in Expression (3B) increases as the edema progresses in one of the upper right limb PAR and the upper left limb PAL than in the other.

As understood from the foregoing description, the index calculating section 44 of the second embodiment functions as an element for calculating the edema index value E according to the discrepancy between the body composition index value (CAR or CBR) based on the bioelectric impedance Z (ZAR or ZBR) of the right limb (upper right limb PAR or lower right limb PBR) and the body composition index value (CAL or CBL) based on the bioelectric impedance Z (ZAL or ZBL) of the left limb (upper left limb PAL or lower left limb PBL) of the human subject H.

FIG. 6 is a graph showing the average edema index value E calculated according to Expression (3B), described earlier, for the lower limbs of a plurality of edema patients clinically classified into the edema stages. In the second embodiment, as in the first embodiment, it is understood from FIG. 6 that there is tendency that the larger the edema index value E is , the higher the edema stage is (the more the edema has progressed).

The edema evaluation section 46 of the second embodiment identifies the edema stage corresponding to the edema index value E calculated by the index calculating section 44 among a plurality of edema stages (0, I, IIa, IIb, and III). How the edema stage is identified is the same as in the first embodiment. Specifically, the edema evaluation section 46 compares reference values Q (Q0, Q1, Q2, and Q3) stored in the storage unit 12 with the edema index value E to identify the edema stage corresponding to the edema index value E. Since the edema index value E in the second embodiment differs in value ranges from the edema index value E in the first embodiment, the reference values Q used by the edema evaluation section 46 of the second embodiment to identify the edema stage are different from the reference values Q used in the first embodiment.

As in the first embodiment, it is possible to include a healthy condition in the result of evaluation performed by the edema evaluation section 46. The result reporting section 48 causes the display unit 14 to display an evaluation result image 52 that provides the edema index value E calculated by the index calculating section 44 and the edema stage identified by the edema evaluation section 46.

The second embodiment also achieves the same advantages as the first embodiment. In the second embodiment, since the edema index value E is calculated according to the discrepancy between the body composition index value (CAR or CBR) of a right limb and the body composition index value (CAL or CBL) of a left limb of the human subject H, the user can easily recognize the difference in the degree of progress of the edema between the right and left limbs. In addition, the difference of the body composition index value C between the right and left limbs is divided by the weight W of the human subject H, so that differences in body frame (weight) among human subjects are corrected for and the level of edema can be accurately evaluated.

**Third embodiment**

In the second embodiment, the edema index value E is calculated according to the difference in the body composition index value between the right and left limbs. Therefore, when edema occurs in both the right and left limbs of the human subject H (especially when the levels of edema in the right and left limbs are close), the edema index value E is small (in other words, is close to that obtained when the levels of edema are lower in both the right and left limbs). Taking this situation into account, in a third embodiment, the level of edema is evaluated separately in the right and left limbs when edema occurs in both the right and left limbs of the human subject H.

The index calculating section 44 of the third embodiment can execute a first process in which the edema index value E is calculated for each portion of the body of the human subject H, as in the first embodiment, and a second process in which the edema index value E is calculated according to the difference in the body composition index value C between right and left limbs, as in the second embodiment. In addition, as in the second embodiment, the user can operate the console unit 16 appropriately to specify either upper limbs or lower limbs of the body of the human subject H as target portions. In the following description, however, a case in which the user specifies upper limbs as target portions is described for the sake of convenience.

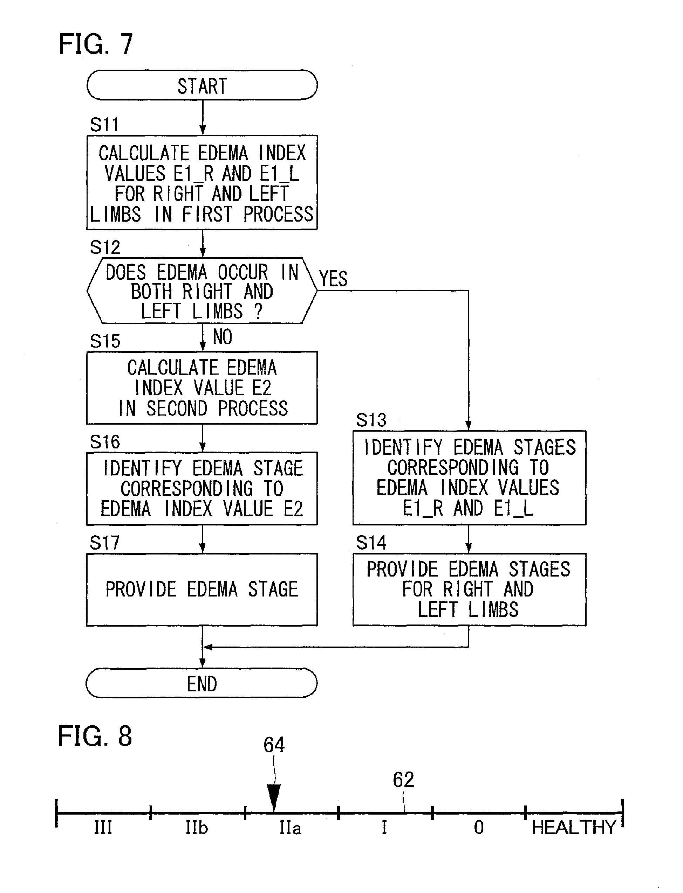

FIG. 7 is a flowchart of an edema evaluation process in the third embodiment. The edema evaluation process shown in FIG. 7 is executed every time the user gives the console unit 16 an instruction to start measurement. When the edema evaluation process starts, the index calculating section 44 executes the first process to individually calculate an edema index value E1_R according to the bioelectric impedance ZAR of the upper right limb PAR and an edema index value E1_L according to the bioelectric impedance ZAL of the upper left limb PAL of the human subject H (S11). How the edema index values E (E1_R and E1_L) are calculated for the portions in the first process is the same as in the first embodiment (Expression (1)).

The index calculating section 44 determines (S12) whether edema occurs in both the upper right limb PAR and the upper left limb PAL of the human subject H according to the edema index values E1_R and E1_L calculated in step S 11. More specifically, the index calculating section 44 compares the edema index values E1_R and E1_L with a predetermined threshold ETH, and, when both the edema index values E1_R and E1_L exceed the predetermined threshold ETH, determines that edema occurs in both the upper right limb PAR and the upper left limb PAL.

When the determination result is affirmative (Yes in step S12), the edema evaluation section 46 identifies the edema stages corresponding to the edema index values E1_R and E1_L calculated in the first process (S13). Specifically, the edema evaluation section 46 compares the edema index value E1_R with each of the reference values Q to identify the edema stage of the upper right limb PAR and also compares the edema index value E1_L with each of the reference values Q to identify the edema stage of the upper left limb PAL. Then, the result reporting section 48 causes the display unit 14 to display an evaluation result image 52 for each of the upper right limb PAR and the upper left limb PAL in the same manner as in the first embodiment (S14). In other words, the edema stage of the upper right limb PAR and the edema stage of the upper left limb PAL are individually provided.

In contrast, when edema occurs in either the upper right limb PAR or the upper left limb PAL, or when edema occurs in neither the upper right limb PAR nor the upper left limb PAL, the determination result in step S12 is negative. When the determination result is negative (No in step S12), the index calculating section 44 executes the second process to calculate the edema index value E2 according to the discrepancy between the body composition index value CAR of the upper right limb PAR and the body composition index value CAL of the upper left limb PAL (S 15). The edema index value E corresponding to the difference in the right and left limbs of the body of the human subject H is calculated in the second process in the same way as in the second embodiment (Expression (3A) or Expression (3B)).

When the edema index value E2 is calculated in the second process, the edema evaluation section 46 identifies the edema stage corresponding to the edema index value E2 (S16). Specifically, the edema evaluation section 46 compares the edema index value E2 calculated in the second process with each of the reference values Q stored in the storage unit 12 to identify the edema stage corresponding to the edema index value E2. Then, the result reporting section 48 causes the display unit 14 to display an evaluation result image 52 that includes the edema stage of the upper limb of the human subject H, in the same manner as in the second embodiment (S 17).

In the foregoing description, the upper limbs of the human subject H were specified as target portions. Even when the lower limbs of the human subject H are specified as target portions, the same edema evaluation processing is executed. As understood from the above description, comprehensively speaking, the first process individually calculates the edema index value E1_R of a right limb (upper right limb PAR or lower right limb PBR) and the edema index value E1_L of a left limb (upper left limb PAL or lower left limb PBL) of the human subject H, and the second process calculates the edema index value E2 according to the discrepancy between the body composition index value of the right limb and the body composition index value of the left limb. When edema occurs in both a right limb and a left limb of the human subject H, the edema evaluation section 46 individually identifies the edema stage corresponding to the edema index value E1_R and the edema stage corresponding to the edema index value E1_L; and when edema occurs in either a right limb or a left limb, the edema evaluation section 46 identifies the edema stage corresponding to the edema index value E2 calculated in the second process.

The third embodiment also achieves the same advantages as the first and second embodiments. In the third embodiment, when edema occurs in either a right limb or a left limb of a human subject H, the edema stage is identified from the edema index value E based on the difference in body composition index value C between the right and left limbs; and when edema occurs in both a right limb and a left limb of the human subject H, the calculation of the edema index value E and the identification of the edema stage are performed individually for the right and left limbs. Therefore, an advantage is provided in that the evaluation result of the edema of the human subject H can be provided appropriately for either a case in which edema occurs in either a right limb or a left limb or a case in which edema occurs in both a right limb and a left limb.

**Modifications**

The embodiments described above can be modified in various ways. Specific example modifications will be described below. Two or more of the following modifications selected in a desired manner can be appropriately combined.

(1) The method for calculating an edema index value E according to the bioelectric impedance Z is not limited to those described in the above embodiments. For example, lean body mass or muscle mass may be calculated from the bioelectric impedance Z with a known method as the edema index value E, or the edema index value E may be calculated according to the ratio of the reactance X to the resistance R ($|X|/R$) of the bioelectric impedance Z. Alternatively, the bioelectric impedance Z itself can be used as the edema index value E (therefore, the computational processor 444 is removed from the index calculation section 44). Basic information (for example, the weight W, the height L, the age, the gender, and so forth) of the human subject H can be reflected in the edema index value E. Any body composition index value, such as lean body mass (for example, a value calculated from the bioelectric impedance Z), can be used as the basic information. As understood from the above description, comprehensively speaking, the edema index value E is a variable serving as a scale for the level of edema (the amount of water), calculated according to the bioelectric impedance Z, and any method can be used to calculated the edema index value E.

(2) The contents of the evaluation result image 52, which reports the edema stage to the user, are not limited to those described above. For example, as shown in FIG. 8, it is possible to display, on the display unit 14, an evaluation result image 52 in which an indicator (cursor) 64 is disposed at the position corresponding to the edema index value E calculated by the index calculating section 44, on a straight-line-shaped image 62 representing the number line of the edema index value E and divided into edema stages. Even with the evaluation result image 52 shown in FIG. 8, the user can easily recognize both the edema index value E calculated by the index calculating section 44 and the edema stage identified by the edema evaluation section 46. The method of reporting used by the result reporting section 48 is not limited to displaying an image. For example, it is possible to cause a printing section that prints an image on paper to print the evaluation result image 52.

(3) The method of using the evaluation results (the edema index value E and the edema stage) is not limited to those described above. For example, it is possible to add a function for displaying or comparing evaluation results obtained at points in time (dates and times) specified by the user (calendar function), a function for displaying temporal changes in evaluation results in a graph, or a function for sending the evaluation results to a server apparatus installed at a hospital to allow medical staff to monitor the level of edema of the  human subject H (and further to propose a self care plan based on the level of edema of the human subject H).

(4) In the second embodiment, the difference in body composition index value C between right and left limbs is divided by the weight of the human subject H to calculate the edema index value E. The divisor of the difference in body composition index value C between right and left limbs is not limited to the weight of the human subject H. For example, differences in body frame among human subjects H can be corrected for even when the difference in body composition index value C between right and left limbs is divided by a body frame index value (such as the lean body mass or the muscle mass) reflecting the body frame of the human subject H. As understood from the above description, it is preferable that the difference between the body composition index value (CAR or CBR) of a right limb and the body composition index value (CAL or CBL) of a left limb of the human subject H be divided by a body frame index value (such as the weight, the lean body mass, or the muscle mass) of the human subject H. Any body frame index value can be used.

## Description of Reference Numerals

| | |
|---|---|
| 100: | Edema evaluation apparatus |
| 10: | Controller |
| 12: | Storage unit |
| 14: | Display unit |
| 16: | Console unit |
| 20: | Group of electrodes |
| 22 (22AR, 22AL, 22BR, and 22BL): | Current supplying electrodes 24 (24AR, 24AL, 24BR, and 24BL): Voltage measuring electrodes |
| 30: | Electrode controller |
| 32: | Current supplying unit |
| 34: | Voltage measuring unit |
| 42: | Basic-information acquisition section |
| 44: | Index calculating section |
| 442: | Measurement processor |
| 444: | Computational processor |
| 46: | Edema evaluation section |
| 48: | Result reporting section |
| 52: | Evaluation result image |

## Claims

1.  An edema evaluation apparatus comprising:

   index calculator for calculating an edema index value indicating the level of edema at a target portion of the body of a human subject, according to a bioelectric impedance of the target portion;
   edema evaluator for identifying an edema stage corresponding to the edema index value calculated by the index calculator from among a plurality of edema stages indicating the degrees of progress of edema symptoms;

and
result reporter for reporting the edema stage identified by the edema evaluator.

2.  The edema evaluation apparatus according to Claim 1, wherein the result reporter further reports the edema index value calculated by the index calculator.

3.  The edema evaluation apparatus according to Claim 1 or 2, wherein the index calculator calculates the edema index value according to a discrepancy between a body composition index value corresponding to a bioelectric impedance of a right limb and a body composition index value corresponding to a bioelectric impedance of a left limb of the human subject.

4.  The edema evaluation apparatus according to Claim 3, wherein the index calculator calculates the edema index value by dividing the difference between the body composition index value of the right limb and the body composition index value of the left limb of the human subject by a body frame index value of the human subject.

5.  The edema evaluation apparatus according to any of Claims 1 to 4, wherein the index calculator can execute a first process in which an edema index value corresponding to a bioelectric impedance of a right limb and an edema index value corresponding to a bioelectric impedance of a left limb of the human subject are individually calculated, and a second process in which an edema index value is calculated according to a discrepancy between a body composition index value corresponding to a bioelectric impedance of a right limb and a body composition index value corresponding to a bioelectric impedance of a left limb of the human subject; and
    when edema occurs in both the right limb and the left limb of the human subject, the edema evaluator individually identifies, for each of the right and left limbs, the edema stage corresponding to the edema index value calculated in the first process, and when edema occurs in either the right limb or the left limb, the edema evaluator identifies the edema stage corresponding to the edema index value calculated in the second process.

6.  The edema evaluation apparatus according to Claim 5, wherein the index calculator determines whether edema occurs in both the right limb and the left limb of the human subject, according to the edema index values calculated in the first process for the right and left limbs, and when edema occurs in either the right limb or the left limb, the index calculator executes the second process.

# FIG. 1

FIG. 2

Dv

442 — MEASUREMENT PROCESSOR — 44

Z

444 — COMPUTATIONAL PROCESSOR ← L

E

FIG. 3

FIG. 4

| EDEMA STAGE | EDEMA INDEX VALUE E |
|---|---|
| HEALTHY | $E \leq Q_H$ |
| 0 | $Q_H < E \leq Q_0$ |
| I | $Q_0 < E \leq Q_1$ |
| IIa | $Q_1 < E \leq Q_2$ |
| IIb | $Q_2 < E \leq Q_3$ |
| III | $Q_3 < E$ |

# FIG. 5

LAST TIME     THIS TIME

110 ——→ **100** ↘

EDEMA STAGE IIa

IMPROVED

—52

# FIG. 6

## FIG. 7

START

S11
CALCULATE EDEMA INDEX
VALUES E1_R AND E1_L
FOR RIGHT AND LEFT
LIMBS IN FIRST PROCESS

S12
DOES EDEMA OCCUR IN
BOTH RIGHT AND
LEFT LIMBS ?  —— YES

NO

S15
CALCULATE EDEMA
INDEX VALUE E2
IN SECOND PROCESS

S16
IDENTIFY EDEMA STAGE
CORRESPONDING TO
EDEMA INDEX VALUE E2

S17
PROVIDE EDEMA STAGE

S13
IDENTIFY EDEMA STAGES
CORRESPONDING TO
EDEMA INDEX VALUES
E1_R AND E1_L

S14
PROVIDE EDEMA STAGES
FOR RIGHT AND
LEFT LIMBS

END

## FIG. 8

64

62

| III | IIb | IIa | I | 0 | HEALTHY |

Europäisches
Patentamt

European
Patent Office

Office européen
des brevets

# EUROPEAN SEARCH REPORT

Application Number

EP 13 00 1338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/002992 A1 (IMPEDANCE CARDIOLOGY SYSTEMS I [US]; CHETHAM SCOTT [AU]) 11 January 2007 (2007-01-11) * figures 3, 7, 8A, B * * page 2, lines 21-27 * * page 4, lines 11-13 * * page 16, lines 2-5 * * page 18, lines 10-13 * * page 21, lines 5-6 * * page 27, lines 3-7 * * page 28, lines 25-29 * * page 31, lines 1-8 * * page 32, lines 4-8 * * page 33, lines 1-10 * ----- | 1-6 | INV. A61B5/053 A61B5/00 |
| X | EP 1 132 046 A1 (YAMATO SCALE CO LTD [JP]) 12 September 2001 (2001-09-12) * figures 3-5 * * paragraphs [0009], [0011], [0015], [0016] * * paragraphs [0034], [0036], [0039] * * paragraphs [0042], [0072] * ----- | 1-6 | |
| A | EP 1 063 500 A2 (TANITA SEISAKUSHO KK [JP]) 27 December 2000 (2000-12-27) * paragraphs [0039], [0044] * ----- | 1-6 | TECHNICAL FIELDS SEARCHED (IPC)  A61B |

The present search report has been drawn up for all claims

| Place of search | Date of completion of the search | Examiner |
|---|---|---|
| The Hague | 24 June 2013 | Almeida, Mariana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 00 1338

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2013

| Patent document cited in search report | | Publication date | Patent family member(s) | | Publication date |
|---|---|---|---|---|---|
| WO 2007002992 | A1 | 11-01-2007 | AU<br>CA<br>EP<br>EP<br>JP<br>JP<br>US<br>WO | 2006265762 A1<br>2613524 A1<br>1903938 A1<br>2250963 A2<br>5034028 B2<br>2008546509 A<br>2009143663 A1<br>2007002992 A1 | 11-01-2007<br>11-01-2007<br>02-04-2008<br>17-11-2010<br>26-09-2012<br>25-12-2008<br>04-06-2009<br>11-01-2007 |
| EP 1132046 | A1 | 12-09-2001 | AU<br>AU<br>CA<br>CN<br>EP<br>JP<br>TW<br>US<br>WO | 757258 B2<br>6726900 A<br>2348601 A1<br>1327376 A<br>1132046 A1<br>4597450 B2<br>529930 B<br>6643542 B1<br>0115600 A1 | 13-02-2003<br>26-03-2001<br>08-03-2001<br>19-12-2001<br>12-09-2001<br>15-12-2010<br>01-05-2003<br>04-11-2003<br>08-03-2001 |
| EP 1063500 | A2 | 27-12-2000 | CN<br>DE<br>DE<br>EP<br>EP<br>EP<br>EP<br>EP<br>KR<br>KR<br>TW<br>US<br>US | 1277007 A<br>60014509 D1<br>60014509 T2<br>1063500 A2<br>1464277 A2<br>1464278 A2<br>1464279 A2<br>1464280 A2<br>20010007331 A<br>20020067687 A<br>514510 B<br>RE42833 E1<br>6487445 B1 | 20-12-2000<br>11-11-2004<br>13-10-2005<br>27-12-2000<br>06-10-2004<br>06-10-2004<br>06-10-2004<br>06-10-2004<br>26-01-2001<br>23-08-2002<br>21-12-2002<br>11-10-2011<br>26-11-2002 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010526604 A